# FASCICULE DE BREVET EUROPEEN

(11) **EP 0 621 007 B1**
(45) Date de publication et mention de la délivrance du brevet: **26.11.1997**
(21) Numéro de dépôt: 94400868.9
(22) Date de dépôt: 21.04.1994
(51) Int. Cl.: A61B 17/12, A61B 17/04

(54) **Système pour ligature et/ou suture pour chirurgie endoscopique**
Abbindungs-und/oder Nähsystem für die endoskopische Chirurgie
Ligaturing and/or suturing system for endoscopic sugery

(30) Priorité: 23.04.1993 FR 9304839
(43) Date de publication de la demande: 26.10.1994
(73) Titulaire: ETHNOR, F-92200 Neuilly sur Seine (FR)
(72) Inventeur: Leroy, Joel, F-62160 Bully-Les-Mines (FR); Catteau, Gilles, F-28630 Barjouville (FR)
(74) Mandataire: Martin, Jean-Jacques

(56) Documents cités:
- EP-A- 0 477 020
- WO-A-92/11810
- DE-A- 2 804 070
- DE-A- 3 504 202
- DE-U- 9 204 296
- FR-A- 736 756
- SU-A- 1 251 885
- US-A- 2 610 631
- US-A- 3 476 115
- US-A- 4 235 238
- US-A- 5 201 744

## Description

La présente invention concerne le domaine des instruments pour chirurgie endoscopique.

La présente invention concerne plus précisément les instruments pour chirurgie endoscopique adaptés pour ligaturer ou suturer un organe.

La chirurgie endoscopique englobe toutes les opérations qui sont réalisées en introduisant les instruments dans l'organisme par les voies naturelles ou par de miniscules incisions et avec examen de l'intérieur du corps à l'aide d'un appareil optique introduit dans l'organisme par les mêmes moyens.

On sait que la chirurgie endoscopique offre de nombreux avantages par rapport aux techniques chirurgicales classiques selon lesquelles le chirurgien opère à corps ouvert.

On peut en particulier citer les avantages suivants :
- la chirurgie endoscopique supprime les cicatrices,
- elle est plus confortable pour le patient et entraîne moins de douleur,
- elle conduit à une plus faible consommation de médicaments d'appoint, notamment des médicaments antalgiques-antidouleurs,
- elle permet une cicatrisation plus rapide,
- elle autorise un rétablissement plus rapide et par conséquent des séjours hospitaliers plus courts, notamment du fait que les complications opératoires et post-opératoires liées à l'ouverture du corps dans les techniques classiques, disparaissent.

Dans ces conditions, on estime ajourd'hui que dans les dix ans qui viennent, les 3/4 des interventions gynécologiques ou liées au système digestif seront réalisées par chirurgie endoscopique.

De nombreux instruments miniaturisés ont été proposés à cet effet, et en particulier des pinces, ciseaux, palpateurs, bistouris électriques ... Tous ces instruments miniaturisés sont conçus pour être introduits dans le corps, par la voie de trocarts de faible section.

Dans le domaine particulier des ligatures ou sutures pour chirurgie endoscopique, les chirurgiens disposent de diverses techniques.

Celles-ci sont présentées par exemple dans le document British Medical Bulletin (1986) vol. 42, n° 3, pp284-295 "Operative Pelviscopy" K. Semm.

La première technique dite de ligature à boucle utilise un fil prémuni en extrémité d'une boucle ou lasso à noeud coulissant. Pour utiliser cette ligature la boucle est introduite dans la cavité du corps, par un trocart, engagée sur l'organe à ligaturer, puis serré sur celui-ci. Le serrage de la boucle peut être opéré à l'aide d'un instrument pousse noeud.

On peut pour cela utiliser différents instruments comportant une tige rectiligne adaptée pour être engagée dans le trocart et comportant à son extrémité proximale une gorge ou fourche par laquelle le pousse noeud est engagé sur le fil et qui sert d'appui au noeud coulissant, pour serrer la boucle, lors d'un déplacement relatif pousse noeud/fil. On a décrit des exemples de tels pousse-noeuds classiques à gorge ou fourche dans les documents 1) Obstetrics & Gynecology, vol. 78, n° 1, Juillet 1991 "A New Clinch Knot" Peter V. Weston et 2) Obstetrics & Gynecology vol. 79, n° 1, Janvier 1992 "A simple method for ligating with straight and curved needles in operative laparoscopy" Harry Reich et al.

Les pousse-noeuds à fourche ou gorge latérale présentent un inconvénient majeur: ils entraînent une torsion du fil et de la boucle sur son axe ; pour maintenir le noeud dans la gorge ou la fourche il est en effet nécessaire de faire passer le fil autour de l'axe du pousse-noeud. Ce phénomène de torsion de la boucle est d'autant plus important que le second est serré et il peut alors entraîner un serrage insuffisant de la boucle.

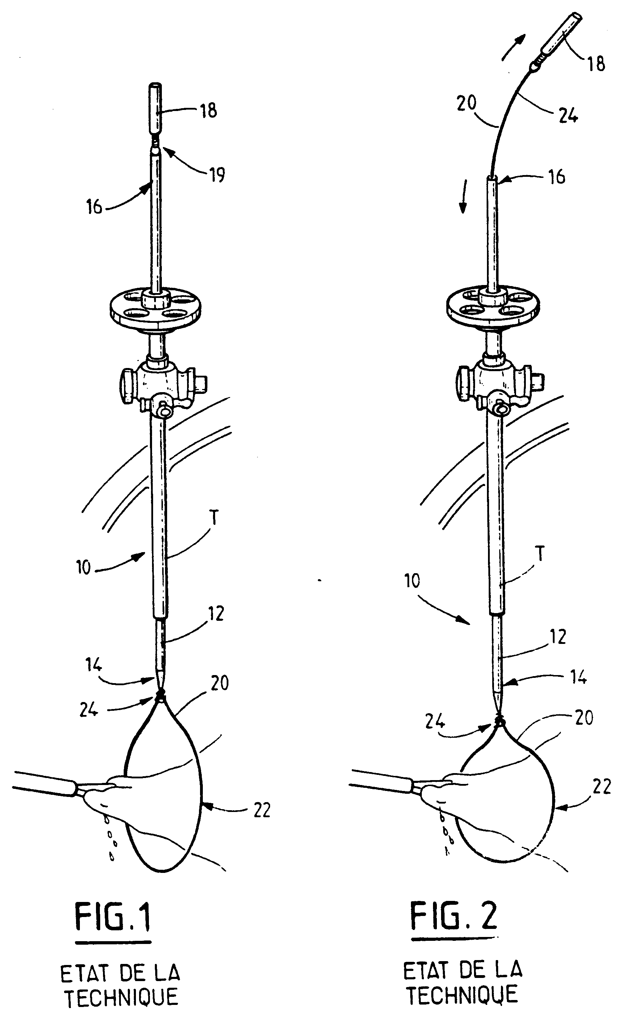

Pour faciliter l'opération de ligature, on a également proposé, comme illustré sur les figures 1 et 2 annexées un pousse noeud 10 comportant un manchon 12 allongé rectiligne adapté pour être engagé dans un trocart T et possédant un canal longitudinal dans lequel est engagé le fil de ligature 20. Ce dernier émerge à l'extrémité proximale 14 du manchon 12 sous forme de la boucle 22 à noeud coulissant 24. De plus, le manchon 12 est muni à son extrémité distale 16 d'un embout 18 fixé sur l'extrémité distale 24 du fil 20. A l'origine l'embout 18 est relié au manchon 12 par une zone de faiblesse 19. Pour utiliser ce pousse noeud 10, il suffit, après avoir engagé la boucle 22 sur l'organe à ligaturer (comme représenté sur la figure 2), de séparer l'embout 18 et le manchon 12 au niveau de la zone de faiblesse 19 et de déplacer ces derniers en éloignement pour serrer la boucle 22.

Une seconde technique consiste à réaliser la boucle de ligature autour de l'organe au moment de l'intervention. Dans ce cas, le noeud coulissant peut être réalisé de façon entièrement intracorporelle, par manipulation du fil à l'intérieur de la cavité du corps, ou de façon extracorporelle en ressortant le brin du fil passé autour de l'organe, par le trocart, en formant le noeud coulissant à l'extérieur du corps et en introduisant le noeud dans le trocart et la cavité du corps pour serrer la ligature. Là encore, un pousse noeud doit être utilisé généralement, au moins dans le cas de réalisation de noeuds extracorporels. On peut pour cela utiliser des pousse-noeuds à gorge ou fourche classiques comme indiqué précédemment. Les chirurgiens disposent également aujourd'hui de fils à ligaturer montés sur des pousse-noeuds à manchon et embout séparables du type représenté sur les figures 1 et 2.

Une troisième technique consiste à utiliser un fil de suture, c'est-à-dire un fil muni d'une aiguille, et non pas un fil de ligature. Là encore, le noeud coulissant peut être réalisé en intracorporel ou en extracorporel. La suture peut être serrée à l'aide d'un pousse-noeud classique à gorge ou fourche. Les chirurgiens disposent également de fils de suture montés sur des pousse-noeuds à manchon et embout séparables du type représenté sur les figures 1 et 2.

Les documents EP-A-477020 et DE-U-9204296 enseignent des exemples de dispositif du type illustré sur les figures 1 et 2 annexées.

Le document US-5201744 enseigne un exemple de pousse noeud.

Le document WO-A-92/11810 décrit un autre système pour ligature endoscopique qui comprend un fil prémuni d'une boucle coulissante placé sur un support allongé. Ce système est d'utilisation très limitée. Il crée inévitablement un cisaillement sur l'organe à ligaturer lors du serrage de la boucle. Par ailleurs, il impose un conditionnement volumineux.

Les diverses techniques pour ligature et/ou suture, rappelées ci-dessus sont utilisées de nos jours par les chirurgiens.

Toutefois, ces techniques ne donnent pas totalement satisfaction.

En particulier, la manipulation des pousse-noeud à fourche ou gorge, décrits par exemple dans les documents Obstetrics & Gynecology cités ci-dessus, est assez délicate.

Quant aux pousse-noeuds à manchon et embout séparables du type représenté sur les figures 1 et 2 annexées, ils présentent l'inconvénient d'exiger un emballage volumineux adapté à la longueur du manchon. Et cet inconvénient est d'autant plus pénalisant que le chirurgien ne pouvant bien souvent décider qu'au tout dernier moment la nature du fil et le diamètre du fil, voir le type d'aiguille, utilisé pour ligaturer ou suturer, il est nécessaire de disposer dans la salle d'opération d'une gamme complète d'ensembles comprenant un pousse-noeud à manchon et embout séparables équipés de fils de ligature ou de sutures de différents diamètres, de différentes natures et pourvues, pour les fils de sutures d'aiguilles de géométrie diverses, telles que des aiguilles droites, des aiguilles ski etc ...Cela conduit à un volume impressionnant pour les seuls ligatures et sutures. Par ailleurs, il est à noter que les dimensions des emballages requis pour ces pousse-noeuds à manchon et embout séparables rendent les opérations de stérilisation assez couteuses.

La présente invention a maintenant pour but de perfectionner les systèmes de ligature et suture existants pour chirurgie endoscopique.

Un but important de la présente invention est de réduire le volume des emballages stériles requis pour les ligatures et/ou sutures.

Un autre but important de la présente invention est de proposer un nouveau système de ligature ou suture permettant de simplifier la manipulation au cours de l'opération chirurgicale.

Ces buts sont atteints dans le cadre de la présente invention, grâce à un système du type définé en revendication 1 annexée

Comme on le comprendra à la lecture de la description qui va suivre, la présente invention permet de conditionner individuellement les liens équipés d'un embout tubulaire, sous emballages stériles, et ce séparément des supports.

Selon une autre caractéristique avantageuse de la présente invention, l'extrémité distale du lien est pourvue d'un curseur et la poignée de préhension du support est munie d'une glissère complémentaire.

Cette dernière caractéristique permet en particulier de manipuler le système à l'aide d'une seule main.

La présente invention concerne également séparément d'une part, les liens équipés d'un embout tubulaire et d'autre part, les supports associés comportant une poignée de préhension et un support à berceau de réception.

Le document DE-A-3504202 décrit un ensemble à ligaturer pour chirurgie comprenant un applicateur tubulaire allongé et un dispositif de liaison monobloc constitué d'un collier de serrage engagé dans une tête à encliquetage solidaire dudit collier.

Les moyens décrits dans ce document sont très éloignés de ceux de l'invention. Tout d'abord, les moyens du document DE-A-3504202 ne sont pas adaptés pour une intervention de type endoscopique. Ensuite, ce document ne décrit pas un lien pour ligature ou suture monté à coulissement dans le canal longitudinal d'un embout tubulaire séparé comme c'est le cas pour l'invention. Et cette différence est fondamentale : l'invention permet après serrage de la ligature ou suture de bloquer celle-ci à l'aide d'un noeud en utilisant l'embout tubulaire comme pousse noeud. En revanche, le dispositif de liaison décrit dans le document DE-A-3504202 ne permet pas la réalisation d'un tel noeud de blocage. Il exige par conséquent la disposition de moyens de soudure et de sectionnement pour le dispositif de liaison, en extrémité de l'applicateur. Par ailleurs, en raison de la présence indispensable de ces moyens de soudure et de sectionnement en extrémité de l'applicateur, ce dernier ne comprend pas un berceau de réception de la tête du dispositif de liaison à son extrémité proximale, mais comprend seulement une chambre de réception de cette tête, en retrait de cette extrémité proximale. Enfin, comme le montre la figure 3 de ce document l'engagement de l'extrémité libre du lien dans ladite tête nécessite un fil en boucle additionnel. L'utilisation de ce fil additionnel est très délicate.

D'autres caractéristiques, buts et avantages de la présente invention apparaîtront à la lecture de la description détaillée qui va suivre, et en regard des dessins annexés donnés à titre d'exemples non limitatifs et sur lesquels:
- les figures 1 et 2 précédemment décrites illustrent un système conforme à l'état de la technique,
- la figure 3 représente une vue schématique d'un lien conforme à la présente invention
- la figure 4 représente une vue schématique en perspective d'un support conforme à la présente invention,
- la figure 5 représente une vue schématique du système conforme à la présente invention combinant lien et support, en vue partiellement en coupe longitudinale du support,

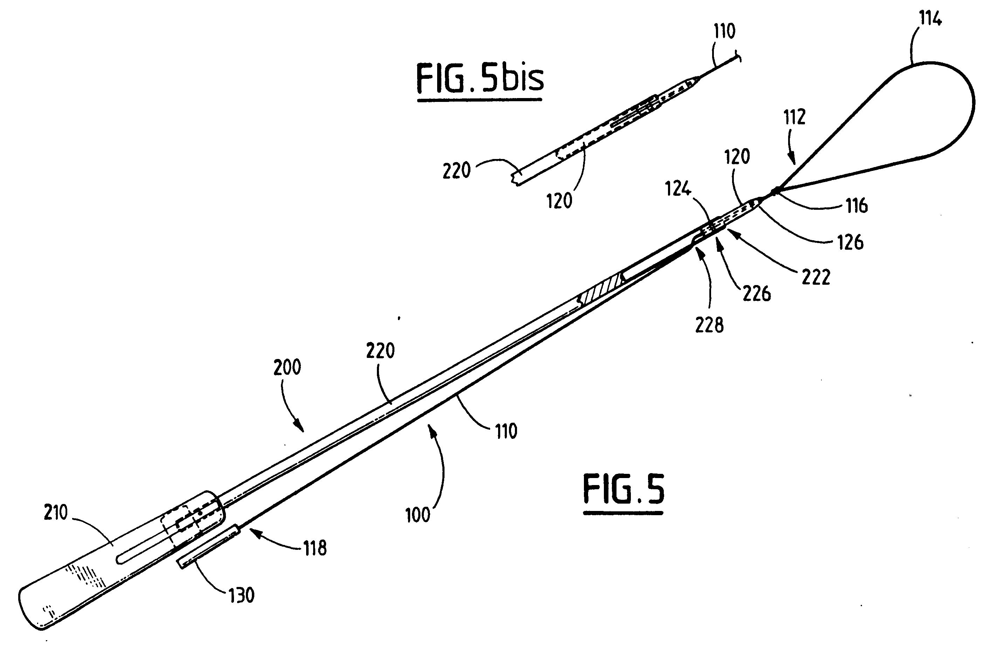

- la figure 5bis représente une vue partielle similaire de l'extrémité proximale du support selon une vue latérale orthogonale à la figure 5,

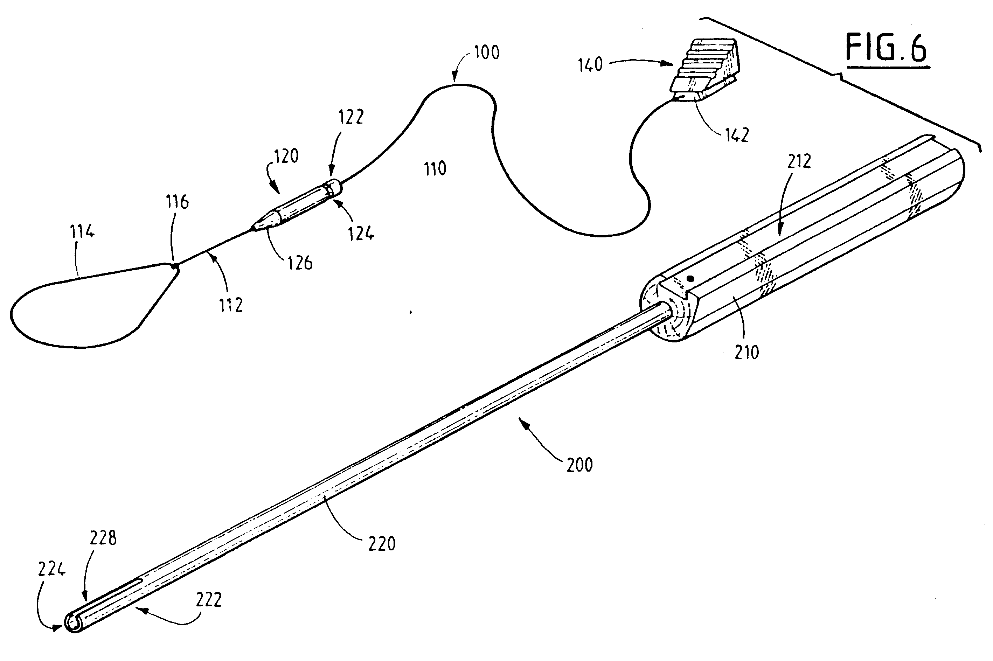

- la figure 6 représente une vue éclatée d'une autre variante de réalisation d'un système conforme à la présente invention, comprenant en combinaison un lien et un support,

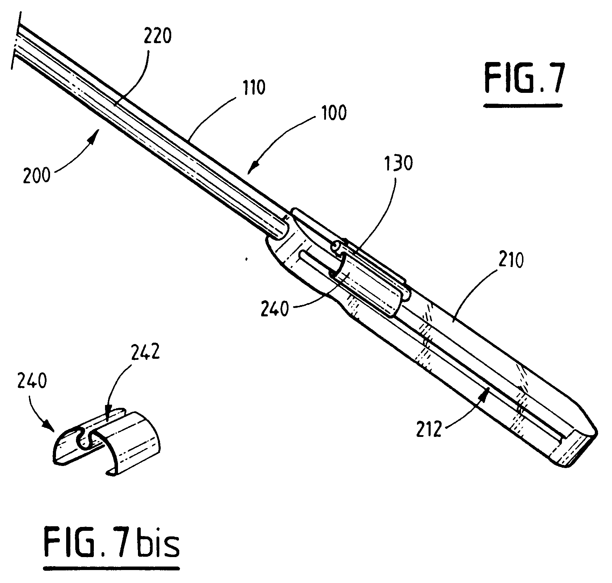

- la figure 7 représente une vue de la poignée d'un support conforme à un autre mode de réalisation de la présente invention,
- la figure 7bis représente une variante de réalisation en perspective d'un curseur conforme à la présente invention,

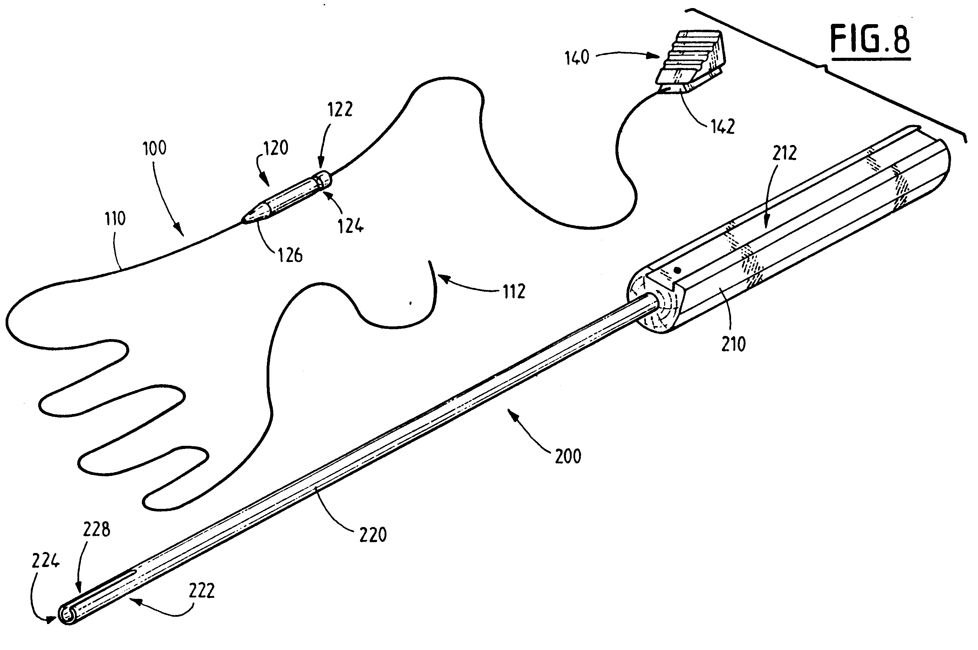

- la figure 8 représente une variante de réalisation conforme à la présente invention relative à une ligature,

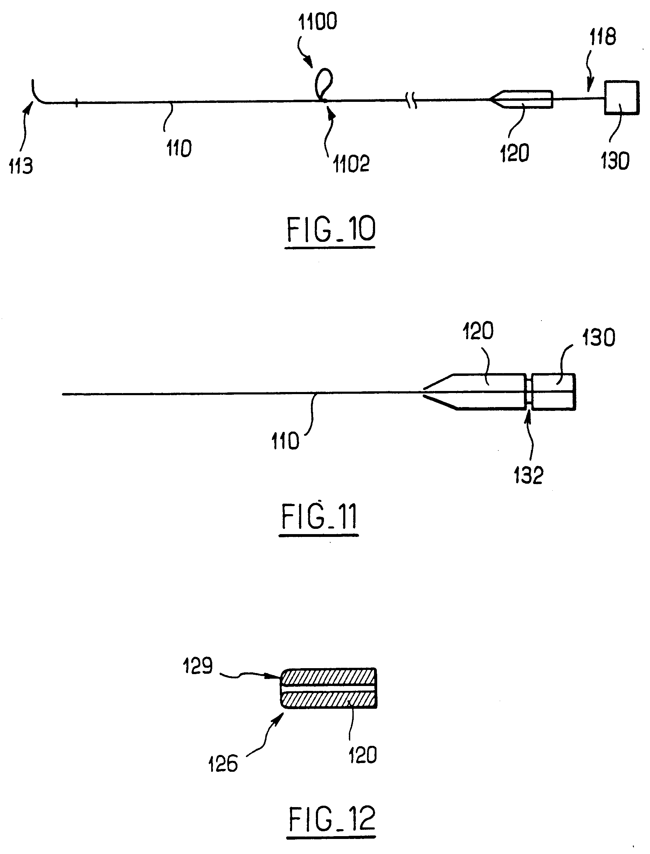

- la figure 9 représente une variante de réalisation d'un embout conforme à la présente invention,
- la figure 10 représente une variante de réalisation de lien conforme à la présente invention,
- la figure 11 représente une autre variante de réalisation comprenant un embout tubulaire provisoirement solidaire d'un embout auxiliaire, et
- la figure 12 représente une vue en coupe longitudinale d'un embout conforme à la présente invention.

Comme on l'a indiqué précédemment, le système conforme à la présente invention comprend en combinaison un lien 100 et un support 200.

Le lien 100 comprend essentiellement un fil 110 et un embout principal 120 formé d'une pièce distincte du fil 110.

Le fil 110 peut être formé en tout matériau approprié, naturel ou synthétique. Le fil 110 peut être un fil résorbable ou non. Il peut s'agir d'un fil mono ou multibrins, de tout diamètre adéquat.

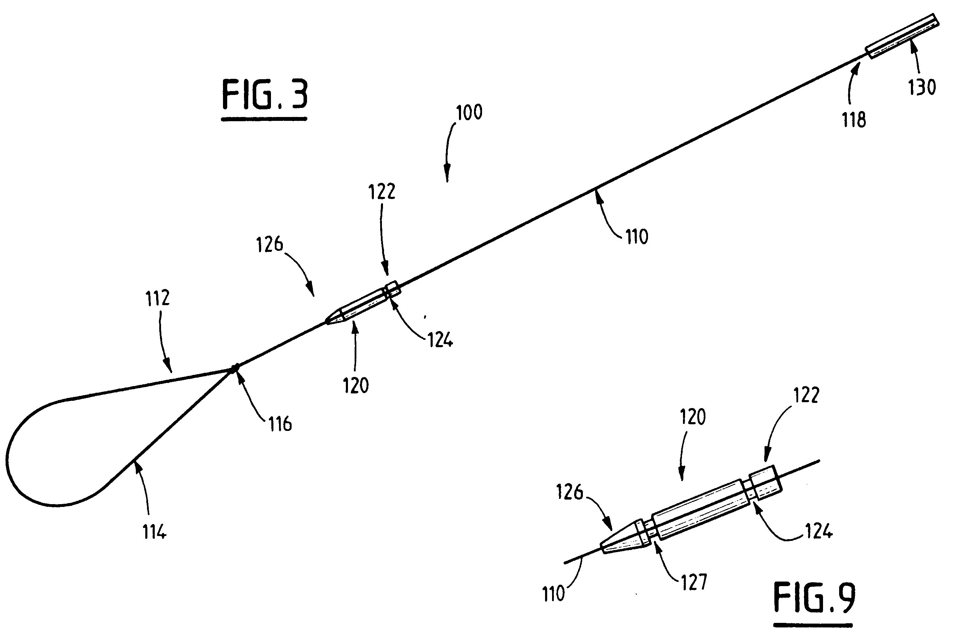

La présente invention s'applique en particulier, mais non exclusivement, au fil de ligature pourvu d'une boucle coulissante. Plus précisément, selon le mode de réalisation particulier représenté sur la figure 3, le fil 110 est pourvu à son extrémité proximale 112 d'une boucle préformée 114 possédant un noeud coulissant 116. Le noeud coulissant 116 peut être de tout type approprié connu de l'homme de l'art. Il autorise le serrage de la boucle 114 par translation du long du brin principal du fil 110.

L'embout tubulaire 120 est pourvu d'un canal longitudinal traversant. Le fil 110 est engagé libre de coulissement dans le canal de l'embout 120. Ainsi, le fil 110 émerge de part et d'autre de celui-ci.

L'embout 120 est réalisé en un matériau stérilisable, tel que par exemple une matière plastique appropriée.

L'embout 120 est adapté pour être monté sur un berceau de réception complémentaire prévu sur le support 200. Ce berceau sera décrit plus en détail par la suite.

A cette fin, l'embout 120 est pourvu de tout moyen approprié pour permettre d'immobiliser ledit embout 120 et le support 200.

Selon le mode de réalisation préférentiel représenté sur la figure 3, l'embout 120 est composé d'un fourreau cylindrique de diamètre extérieur sensiblement constant. A proximité de son extrémité distale 122, l'embout 120 est pourvue, sur sa périphérie extérieure, d'une gorge annulaire 124. Celle-ci présente de préférence une section droite de contour rectangulaire.

De plus au niveau de son extrémité proximale 126, l'embout 120 est de préférence effilé. Ainsi l'extrémité proximale 126 de l'embout 120 est avantageusement tronconique. Cette disposition facilite l'approche d'un organe à ligaturer ou suturer sans risque de traumatisme sur celui-ci.

Le diamètre du canal longitudinal interne de l'embout 120 doit être supérieur au diamètre du fil 110, tout en étant inférieur à l'encombrement transversal du noeud 116, de sorte que celui-ci ne puisse pénétrer dans ledit canal longitudinal de l'embout 120 et que ce dernier serve de pousse-noeud à l'égard du noeud 116.

De préférence, le fil 110 est muni en outre, au voisinage de son extrémité distale 118, d'un embout auxiliaire 130.

Un tel embout auxiliaire 130 a pour but de permettre une reconnaissance ainsi qu'une saisie et une traction manuelle directe sur l'extrémité distale 118 du fil 110 pour faciliter les opérations de ligature. L'embout auxiliaire 130 autorise ainsi une manipulation complète en aveugle, c'est-à-dire sans observation visuelle (par exemple dans l'obscurité) du fil notamment de son extrémité distale 118.

Par ailleurs, l'embout auxiliaire 130 présente une section droite supérieure à celle du canal formé dans l'embout 120. L'embout auxiliaire 130 permet ainsi de garantir que l'extrémité distale 118 n'échappe pas par traversée de l'embout 120.

Enfin, comme on le précisera par la suite, cet embout auxiliaire 130 peut constituer un curseur adapté pour être monté à coulissement sur la poignée 210 du support 200, ou encore être adapté pour être monté sélectivement sur un tel curseur lié en permanence à la poignée 210.

L'embout auxiliaire 130 est réalisé également en un matériau stérilisable, tel que par exemple en matière plastique. L'embout auxiliaire 130 est lié à l'extrémité distale 118 du fil par tout moyen approprié, tel que par exemple par poinçonnement ou sertissage.

L'embout auxiliaire 130 peut faire l'objet de nombreuses variantes de réalisation.

Selon le mode de réalisation particulier représenté sur la figure 3, l'embout auxiliaire 130 a la forme d'un manchon cylindrique de diamètre extérieur constant.

Comme on le verra par la suite, notamment en regard des figures 6 à 8, l'embout auxiliaire 130 peut être conformé en curseur adapté pour être monté à coulissement sur le support 200.

Le lien formé du fil 110, de l'embout principal 120 et le cas échéant d'un embout auxiliaire 130 est conditionné de façon unitaire, dans les conditions ordinaires, sous emballages stériles. Un tel conditionnement classique en lui-même dans le domaine des ligatures et sutures, n'a pas été représenté sur les figures annexées, pour simplifier l'illustration, et ne sera pas précisé par la suite.

On notera cependant, et c'est là un avantage important, qu'un tel conditionnement du lien 100 permet de limiter notablement le volume de stockage par rapport au pousse-noeud connu représenté sur les figures 1 et 2.

Le support 200 conforme à la présente invention comprend essentiellement, d'une pièce, une poignée 210 pourvue d'une tige 220.

Là encore, le support 200 est réalisé avantageusement en un matériau stérilisable. Le support 200 peut être réalisé par exemple en métal, tel qu'à base d'acier inoxydable, ou encore en matière plastique.

Le cas échéant, le support 200 peut être formé par assemblage d'éléments de matières différentes, tels que par exemple par assemblage d'une poignée de préhension 210 en matière plastique sur une tige en métal 220.

La poignée 210 peut faire l'objet de nombreuses variantes de réalisation, quant à sa géométrie et/ou ses dimensions. De nombreux instruments chirurgicaux sont déjà pourvus de poignées de préhension susceptibles d'être retenues dans le cadre de la présente invention. La représentation donnée à cet égard sur les figures annexées, ne doit bien entendu pas être considérée comme limitative. On rappellera simplement que de préférence la poignée de préhension 210 présente une surface extérieure qui n'est pas lisse et cylindrique de révolution, afin de faciliter les manipulations.

La tige 220 est de préférence rectiligne. Elle est pourvue à son extrémité proximale 222 d'un berceau 224 apte à recevoir, de façon amovible, l'embout principal 120 du lien 100.

Le berceau de réception 224 peut également faire l'objet de nombreuses variantes de réalisation.

De préférence, comme cela est représenté sur les figures annexées, le berceau de réception 224 est formé de préférence d'un canal central qui débouche sur l'extrémité axiale proximale de la tige 220.

De préférence, ce canal axial 224 est borgne afin de faciliter les opérations de nettoyage et de stérilisation.

Le diamètre interne du canal 224 servant de berceau de réception doit être adapté au diamètre extérieur de l'embout 120.

En outre, le berceau de réception 224 est pourvu de tout moyen approprié de retenue, permettant de fixer de façon amovible l'embout 120 sur l'extrémité proximale de la tige 220.

Selon le mode de réalisation préférentiel représenté sur les figures annexées, et visible notamment sur les figures 5 et 5bis, la tige 220 est pourvue à cet effet, au niveau de son extrémité proximale, soit en regard du berceau 224, d'une nervure interne 226 complémentaire en dimensions et en positionnement axial de la gorge 124 formée sur l'embout 120.

Bien entendu, on peut prévoir la disposition inverse, c'est-à-dire que l'on peut prévoir une nervure en saillie sur la périphérie extérieure de l'embout 120 et une gorge interne complémentaire dans le berceau 224 de la tige 220.

En outre, selon l'invention, il est prévu sur l'extrémité proximale de la tige 220, une fenêtre 228 latérale traversante par laquelle ledit berceau 224 débouche à l'extérieur de la tige support 220. La fenêtre 228 est de préférence rectiligne et parallèle à l'axe longitudinal de la tige 220.

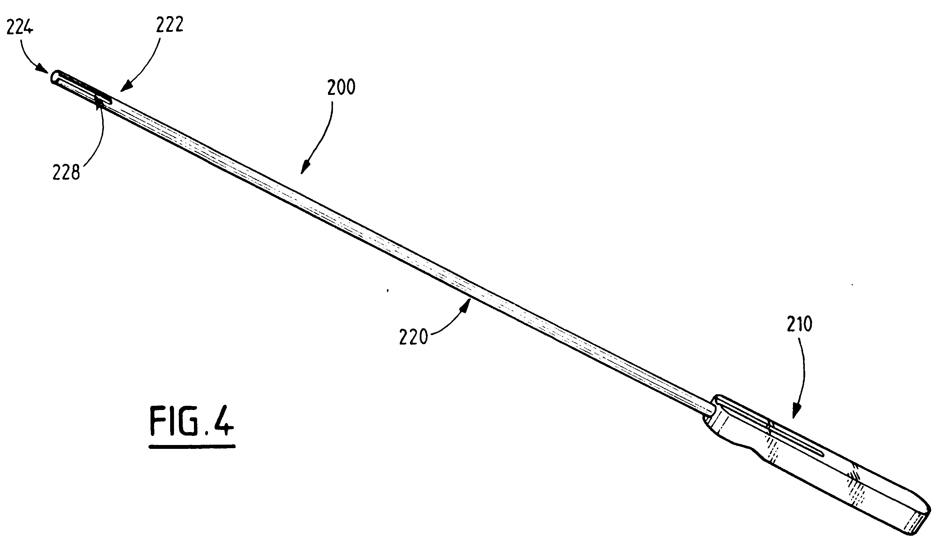

La fenêtre 228 s'étend au moins en regard de l'extrémité distale du berceau 224, soit le fond de celui-ci. Cependant, comme représenté sur la figure 4, la fenêtre 228 peut s'étendre sur toute la longueur dudit berceau 224.

Grâce à la présence de la fenêtre latérale 228, le brin du fil 110 émergeant sur l'extrémité distale de l'embout 120, peut être dégagé sur l'extérieur de la tige 220 lorsque l'embout 120 est placé sur le berceau 224, comme on le voit notamment à l'examen des figures 5 et 5bis.

On comprend par ailleurs que l'existence d'une fenêtre 228 s'étendant sur la totalité de la longueur du berceau 224 permet d'augmenter l'élasticité de celui-ci, et par conséquent de faciliter la mise en place et le retrait de l'embout 120.

De préférence le support 200 est conditionné séparément du lien 100. Par ailleurs, alors que le lien 100 décrit précédemment au regard de la figure 3 est à usage unique, de préférence le support 200 formé de la poignée de préhension 210 de la tige 220, et de préférence ré-utilisable. A cette fin, le support 200 doit bien entendu être stérilisé avant chaque utilisation.

L'utilisation et le fonctionnement du système conforme à la présente invention qui vient d'être décrit est essentiellement le suivant.

Lorsque le chirurgien souhaite réaliser une ligature, il doit tout d'abord choisir le lien 100 approprié en fonction de la nature du fil, et de ses dimensions, et extraire le lien 100 de son emballage stérile.

L'embout 120 du lien 100 est alors positionné sur l'extrémité proximale d'une tige support 220, soit au niveau du berceau 224. Au cours de cette opération, il convient de veiller à passer le brin du fil 110 émergeant sur l'extrémité distale de l'embout 120 par la fenêtre latérale 228.

Dans cet état, la boucle 114 peut être engagée dans un trocart d'intervention, en introduisant l'extrémité proximale 222 de la tige support 220 dans ledit trocart.

Une fois l'organe à ligaturer placé dans la boucle 114, il suffit de tirer sur l'extrémité distale 118 du fil 110, par rapport au support 200 pour serrer la boucle 114 sur l'organe à ligaturer.

Au cours de cette opération, on comprend que l'embout principal 120 tient lieu de pousse-noeud.

Bien entendu, le diamètre externe de la tige support 220 doit être inférieur au diamètre interne du canal longitudinal du trocart associé.

Selon un mode de réalisation particulier donné à titre d'exemple non limitatif, le système répond aux caractéristiques dimensionnelles suivantes:
- longueur de l'embout 120 : 30mm,
- diamètre de l'embout : 4mm,
- diamètre du canal longitudinal interne de l'embout 120 : 0,75mm,
- gorge 124 située à 4mm de l'extrémité distale 122 de l'embout 120,
- largeur de la gorge 124 : 1,5mm,
- profondeur de la gorge 124 : 0,25mm,
- longueur de la partie tronconique proximale 126 de l'embout 120 : 7mm,
- longueur de l'embout auxiliaire 130 : de l'ordre de 15mm,
- diamètre de l'embout auxiliaire 130 : 4mm,
- longueur de la tige support 220 : de l'ordre de 330mm,
- diamètre externe de la tige support 220 : 5mm.

On a représenté sur la figure 6 annexée, une variante de réalisation selon laquelle l'embout auxiliaire 130 prévu à l'extrémité distale 118 du fil 110 est remplacé par un curseur 140. Ce curseur 140 est adapté pour être engagé dans une glissière complémentaire 212 prévue sur la poignée de préhension 210 du support 200. La glissière 212 s'étend de préférence dans une direction générale parallèle à l'axe du support. La glissière 212 est ainsi conçue pour guider à translation axiale le curseur 140 et faciliter le serrage de la boucle 114 sur l'organe à ligaturer.

La gissière 212 et le curseur 140 complémentaire peuvent bien entendu faire l'objet de nombreuses variantes de réalisation.

Selon le mode de réalisation préférentiel conforme à la présente invention, la glissière 212 est formée d'une rainure à bords convergents tels que par exemple une rainure à queue d'aronde. Le curseur 140 possède un tenon 142 de section droite complémentaire de ladite glissière 212.

Bien entendu, en variante, on peut prévoir un tenon longitudinal sur la poignée de préhension 210 et une rainure complémentaire sur le curseur 140.

L'utilisation d'un tel curseur 140 guidé à translation sur la poignée 210, à la place du simple embout auxiliaire 130 représenté sur la figure 3, permet de simplifier la manipulation du système au cours de l'opération chirurgicale. En effet, alors que le mode de réalisation représenté sur les figures 3 à 5 exige deux mains libres : l'une tenant le support 200, l'autre tirant sur l'embout auxiliaire 130, l'utilisation d'un curseur 140 conforme à la figure 6 permet une manipulation du système et le serrage de la boucle 114 à l'aide d'une seule main. En effet, le chirurgien peut aisément tenir la poignée de préhension 210 d'une main et déplacer le curseur 140 à l'aide d'un doigt, par exemple le pouce, de celle-ci.

Selon le mode de réalisation représenté sur la figure 6, le curseur 140 fait partie intégrante du lien 100. Il est fixé sur l'extrémité distale 118 du fil 110. Le curseur 140 est par conséquent conditionné dans l'emballage stérile, avec le lien 110 et l'embout principal 120.

Bien entendu, une fois la ligature achevée, le curseur 140 ainsi que l'embout principal 120 sont éliminés lors du sectionnement du fil superflu.

On a représenté sur la figure 7 annexée, une autre variante de réalisation selon laquelle, il est prévu un curseur 240 ré-utilisable monté a coulissement longitudinal sur la poignée de préhension 210 et adapté pour recevoir l'embout auxiliaire 130 représenté sur la figure 3 lié au fil 110.

A cet effet, le curseur 240 comprend une pince 242 complémentaire dudit embout auxiliaire 130. Un tel curseur 240 peut être formé en tout matériau approprié, par exemple par moulage d'une pièce en matière plastique, ou encore par pliage d'une feuille en matériau métallique, par exemple en acier inoxydable.

Le cas échéant, on peut prévoir un curseur 240 à usage unique. Toutefois, de préférence, le curseur 240 adapté pour recevoir l'embout auxiliaire 130 est monté à demeure sur la poignée de préhension 210 et est de préférence adapté pour être aisément lavé et stérilisé.

Les modes de réalisation qui précèdent concernent des dispositifs du type dit "endoloop" en langage anglo-saxon, c'est-à-dire des systèmes comprenant une boucle 114 à noeud coulissant préformée avant conditionnement sur l'extrémité proximale du fil 110.

Toutefois, comme on l'a schématisé sur la figure 8, la présente invention n'est pas limitée à cette disposition particulière et s'applique également aux liens 110 dépourvus de telle boucle 114 préformée. Il peut s'agir de fil simple pour endoligature avec réalisation de noeuds en intra-corporal ou en extra-corporel, pour lesquels l'embout 120 placé sur la tige support 220 sert de pousse-noeud.

Il peut s'agir encore, dans le cadre de sutures, d'un fil 110 pourvu à son extrémité proximale d'une aiguille de toute géométrie appropriée, là encore avec réalisation de noeuds de blocage en intra-corporel ou en extra-corporel.

Le système conforme à la présente invention offre en particulier les avantages suivants :
- il autorise un encombrement réduit, au stockage, des fils de ligature et/ou de suture 110 équipés de l'embout 120, voire de l'embout auxiliaire 130 ou d'un curseur 140. Il simplifie les opérations de stérilisation au conditionnement,
- il autorise une manipulation à l'aide d'une seule main.

Bien entendu la présente invention n'est pas limitée aux modes de réalisation particuliers qui viennent d'être décrits mais s'étend à toute variante conforme à son esprit.

Ainsi par exemple, on pourra aisément adapter le curseur 240 placé à demeure sur la poignée de préhension 210 selon le mode de réalisation de la figure 7 pour recevoir un simple noeud ou équivalent formé sur l'extrémité distale du fil 110 pour permettre d'exercer une traction sur celui-ci par simple déplacement du curseur 240.

Il faut noter que l'utilisation d'un lien engagé dans un embout tubulaire, comme préconisé par la présente invention, permet d'éviter le phénomène de torsion rencontré avec les pousse-noeuds à fourche ou gorge latérale. En effet, grâce à l'invention, l'un des fils est dans l'axe de poussée. Par ailleurs, l'embout à usage unique peut être parfaitement adapté au fil utilisé alors que cela ne peut être le cas pour un pousse-noeuds à fourche ou gorge latérale à usage multiple.

On peut aussi utiliser l'embout tubulaire 120 conforme à la présente invention, qui est à usage unique, comme un système passe-fil.

Ceci peut être intéressant lorsqu'il faut passer le fil autour de structures assez volumineuses comme le tube digestif ou les gros vaisseaux, car cela peut faciliter le repérage du fil positionné en profondeur autour, par exemple, du rectum dont on veut réaliser la fermeture par une boucle.

Pour utiliser l'embout 120 en tant que passe-fil on peut par exemple déconnecter ledit embout 120 du support rigide 200 et manipuler l'embout 120 à l'aide de pinces classiques.

Pour faciliter le réengagement de l'embout 120 sur un support 200 après avoir contourné la structure, il est avantageux de prévoir, sur l'embout 120 une seconde gorge annulaire 127 a proximité de son extrémité proximale 126 comme représenté sur la figure 9. Cette seconde gorge 127 similaire à la gorge 124 précédemment décrite permet ainsi d'engager l'embout 120, par son extrémité proximale 126, dans le berceau 224 du support rigide 200, et de maintenir l'embout 120 sur le support 200 grâce à la coopération entre la gorge 127 et la nervure 226.

Pour faciliter les manipulations de l'embout 120, on peut également prévoir un support 200 possédant un berceau 224 dont l'axe longitudinal est incliné par rapport à celui du support 200 ou encore un berceau 224 articulé sur l'extrémité proximale 222 du support 200 et associé à des moyens commandables à partir de la poignée 210 pour régler son orientation.

De tels moyens articulés à commande contrôlée sont connus de l'homme de l'art dans le domaine des instruments pour chirurgie endoscopique. Ils ne seront donc pas décrits dans le détail par la suite.

On a représenté sur la figure 10, une variante de réalisation d'une suture conforme à la présente invention comprenant une boucle préformée 1100.

Plus précisément, on aperçoit sur la figure 10, un lien conforme à la présente invention comprenant un fil 110 muni d'une aiguille 113, enagé dans un embout tubulaire 120 et pourvu à son extrémité distale 118 d'un embout auxiliaire 130. Le fil 110 est muni au-delà de l'embout 120, c'est-à-dire entre l'aiguille 113 et cet embout 120 d'une boucle 1100 fermée par un noeud coulissant 1102. Ce lien permet de simplifier la réalisation de ligature puisqu'il suffit de passer l'aiguille 113 dans la boucle préformée 1100 pour obtenir une boucle de ligature autour d'un organe à ligaturer, laquelle boucle de ligature peut être bloquée par fermeture de la boucle préformée 1100 grâce à une traction sur l'embout auxiliaire distal 130.

On a représenté sur la figure 11 une variante de réalisation de lien comprenant un embout tubulaire 120 et un embout auxiliaire 130 venus de moulage et reliés à l'origine, c'est-à-dire avant utilisation, par une zone de faiblesse 132, de plus faible diamètre, pouvant être rompue aisément. Cette variante de la figure 11 peut s'appliquer à l'ensemble des liens de ligature ou de suture, notamment les liens à endoloop du type représenté sur la figure 3, les endoligatures pour noeuds intra ou extracorporels, les liens à aiguille ou encore les liens à boucle préformée du type représenté sur la figure 10.

On a représenté sur la figure 12 une variante d'embout tubulaire 120. Selon la figure 12, l'extrémité proximale 126 de l'embout 120 est non pas conique mais en forme de demi tore 129 centré autour du canal central de l'embout 120. Cette forme en demi tore facilite le décalage des deux brins du fil de lien 110 et facilite les coulissements des noeuds de blocage, en particulier lorsque ceux-ci sont réalisés sous forme de "demi clef" comme cela est classique dans le domaine chirurgical.

Le système conforme à la présente invention permet, dans les versions à endoloop représentées sur la figure 3, d'ouvrir à volonté la dimension de la boucle, ce que ne permet pas le document WO-92/11810 précité.

Il est important dans le cadre de l'invention d'utiliser un embout 120 en matière plastique et un support 200 parfaitement rigide. La rigidité du support facilite la poussée des noeuds par rapport aux documents EP-A-477020 et DE-U-9204296 précités qui enseignent des supports plastiques. De plus, l'embout en matière plastique permet de faciliter le coulissement du lien 110.

Grâce à la possibilité de séparer l'embout 120 du support 200, on peut réaliser grâce à l'invention plusieurs points de ligature indépendants à l'aide de liens différents, avec un support 200 unique.

Il faut noter également que les systèmes antérieurs conduisent tous à un cisaillement sur l'organe à ligaturer lors du serrage de la boucle de ligature. En revanche, la présente invention permet d'éviter ce cisaillement grâce à la possibilité de coulissement du noeud de blocage, notamment lorsqu'il est réalisé sous forme d'une demi clef, par progression le long du brin sur lequel est formé ce noeud et simultanément glissement le long de l'autre brin de sorte que ces deux brins restent sensiblement de même longueur.

Enfin, la présente invention permet de réaliser toutes les techniques de ligature ou suture classiques, y compris tous les types de noeuds connus de l'homme de l'art, que ce soit en intracorporel ou extracorporel.

La présente invention permet également de disposer d'un embout tubulaire de poussée 120 bien adapté à la taille du fil 110, et par conséquent de réaliser des noeuds bien adaptés à ce fil.

La présente invention permet par ailleurs, si cela s'avère nécessaire d'interrompre la réalisation d'une ligature, de débrayer, c'est-à-dire de retirer provisoirement l'embout tubulaire 120 du support 200 tout en disposant de l'embout auxiliaire 130 accessible à l'extérieur du corps en vue d'une reprise de la ligature. Le trocart ainsi libéré peut alors être utilisé pour une intervention auxiliaire. Par exemple si un problème hémorragique est constaté au cours de la réalisation d'une ligature, on peut débrayer l'embout 120 associé à celle-ci du support 200 en veillant à maintenir l'embout auxiliaire correspondant à l'extérieur du corps. Le trocart est ainsi libéré. L'embout 120 d'un second lien adapté peut être placé sur le support 200 et ce second lien peut être ainsi engagé par le trocart pour réalisation d'une hémostase. Après achèvement de celle-ci, le second embout 120 est débrayé et le premier embout 120 est replacé sur le surpport pour achèvement de la ligature. Grâce à la structure de l'invention toutes ces interventions peuvent être réalisées à l'aide d'un trocart unique. Aucun dispositif de la technique antérieure n'offre cette possibilité.

## Revendications

1. Système de ligature et/ou suture pour chirurgie endoscopique, comprenant en combinaison :
- un lien (100) associé à un embout tubulaire (120) comportant un canal longitudinal et susceptible de coulissement dans celui-ci, et
- un support rigide (200) comportant une poignée de préhension (210) solidaire d'une tige allongée (220) laquelle, est pourvue au niveau de son extrémité proximale (222) éloignée de la poignée (210), d'un berceau de réception (224) apte à recevoir, de façon amovible, l'embout tubulaire (120) et d'une fenêtre latérale (228) traversante par laquelle ledit berceau (224) débouche à l'extérieur du support (200) pour permettre le passage du lien (110) de sorte qu'une traction exercée sur une extrémité (118) de celui-ci par rapport au support (200) assure le serrage de la ligature ou de la suture, caractérisé par le fait que :
- l'embout tubulaire (120) est formé d'une pièce séparée, distincte du lien (100), et
- le berceau de réception (224) est formé d'un canal longitudinal qui débouche sur l'extrémité axiale proximale de la tige (220) du support rigide (200).

2. Système selon la revendication 1, caractérisé par le fait que la poignée de préhension (210) du support (200) est munie d'une glissière (212) apte à recevoir à translation un curseur (140, 240) lié à l'extrémité distale (118) du lien (110).

3. Système selon la revendication 2, caractérisé par le fait que le curseur (140) est fixé à demeure sur l'extrémité distale (118) du lien (110).

4. Système selon l'une des revendications 2 ou 3, caractérisé par le fait que le curseur (140) est conditionné avec le lien (100) sous emballage stérile.

5. Système selon la revendication 2, caractérisé par le fait que le curseur (240) est monté à demeure sur la poignée de préhension (210), lequel curseur (240) est adapté pour recevoir de façon amovible l'extrémité distale (118) du lien (110).

6. Système selon la revendication 5, caractérisé par le fait que le curseur (240) comporte une structure de pince (242).

7. Système selon l'une des revendications 1 à 6, caractérisé par le fait que le lien (100) est à usage unique.

8. Système selon l'une des revendications 1 à 7, caractérisé par le fait que le support (200) est ré-utilisable.

9. Système selon l'une des revendications 1 à 8, caractérisé par le fait que l'embout tubulaire (120) est réalisé en matière plastique.

10. Système selon l'une des revendications 1 à 9, caractérisé par le fait qu'il comprend des moyens de retenue aptes à immobiliser sélectivement l'embout tubulaire (120) sur l'extrémité proximale (222) de la tige (220), dans le berceau de réception (224) de celle-ci.

11. Système selon la revendication 10, caractérisé par le fait que les moyens de retenue comprennent au moins une gorge annulaire (124) formée sur l'embout tubulaire (120).

12. Système selon l'une des revendications 1 à 11, caractérisé par le fait que les moyens de retenue comprennent deux gorges annulaires (124, 127) formées sur l'embout tubulaire (120) respectivement au voisinage de ses extrémités proximale et distale.

13. Système selon l'une des revendications 10 à 12, caractérisé par le fait que les moyens de retenue comprennent une nervure (226) formée sur la surface interne du berceau de réception (224).

14. Système selon l'une des revendications 1 à 13, caractérisé par le fait que l'embout tubulaire (120) est effilé à son extrémité proximale (126).

15. Système selon l'une des revendications 1 à 14, caractérisé par le fait que le diamètre interne du canal longitudinal de l'embout tubulaire (120) est supérieur au diamètre du fil (110) composant le lien (100) tout en étant inférieur à l'encombrement transversal d'un noeud coulissant (116) formé sur ce lien (100).

16. Système selon l'une des revendications 1 à 15, caractérisé par le fait que la fenêtre latérale (228) s'étend sur toute la longueur du berceau de réception (224).

17. Système selon l'une des revendications 1 à 16, caractérisé par le fait que l'axe longitudinal du berceau de réception (224) est incliné par rapport à l'axe longitudinal de la tige support (220).

18. Système selon l'une des revendications 1 à 16, caractérisé par le fait que le berceau de réception (224) est articulé sur l'extrémité proximale (222) du support (200) et associé à des moyens de réglage en orientation.

19. Système selon l'une des revendications 1 à 18 prises en combinaison avec la revendication 2, caractérisé par le fait que l'ensemble à glissière (212) et curseur (140, 240) comprend une rainure à bords convergents et un tenon de section droite complémentaire.

20. Système selon l'une des revendications 1 à 19, caractérisé par le fait que le lien (100) est choisi dans le groupe comprenant : un fil de ligature (110) pourvu a son extrémité proximale d'une boucle (114) préétablie à noeud coulissant (116), un fil (110) de ligature simple ou un fil (110) de suture pourvu d'une aiguille à son extrémité proximale.

21. Système selon l'une des revendications 1 à 20, caractérisé par le fait que le fil (110) du lien (100) est pourvu d'un embout auxiliaire (130) à son extremité distale (118).

22. Système selon la revendication 21, caractérisé par le fait que l'embout auxiliaire (130) présente une section droite supérieure à celle du canal traversant l'embout tubulaire (120).

23. Système selon l'une des revendications 21 ou 22, caractérisé par le fait que l'embout auxiliaire (130) est conformé en curseur adapté pour être guidé à coulissement, de façon amovible, sur la poignée de préhension (210).

24. Système selon l'une des revendications 21 ou 22, caractérisé par le fait que l'embout auxiliaire (130) est adapté pour être fixé sur un curseur monté à coulissement sur la poignée de préhension (210).

25. Système selon l'une des revendications 1 à 24, caractérisé par le fait que le lien (110) comprend une boucle préformée (1100) sur sa longueur, entre son extrémité distale et l'embout tubulaire (120).

26. Système selon l'une des revendications 1 à 25, caractérisé par le fait que l'embout tubulaire (120) est venu de moulage avec un embout auxiliaire (130) prévu sur l'extrémité distale du lien, ledit embout tubulaire (120) et l'embout auxiliaire (130) étant reliés à l'origine par une zone de faiblesse (132).

27. Système selon l'une des revendications 1 à 26, caractérisé par le fait que l'extrémité proximale (126) de l'embout tubulaire (120) présente une surface en forme de demi tore (129).

28. Lien pour ligature ou suture dans le cadre d'une opération chirurgicale endoscopique conforme à l'une des revendications 1 à 27, caractérisé par le fait qu'il comprend un lien (100) et un embout tubulaire (120) muni d'un canal longitudinal, formé d'une pièce séparée, distincte du lieu (100), lequel est monté à coulissement dans ledit canal longitudinal, et caractérisé par le fait que ledit embout tubulaire (120) comporte des moyens (124) de fixation amovible sur l'extrémité d'une tige (220) d'un support rigide (200).

29. Support rigide pour opération chirurgicale endoscopique conforme à l'une des revendications 1 à 27, caractérisé par le fait qu'il comporte une poignée de préhension (210) solidaire d'une tige allongée (220), laquelle est pourvue, à son extrémité proximale (222) éloignée de la poignée (210), d'un berceau de réception (224) apte à recevoir de façon amovible un embout tubulaire (120) recevant à coulissement un lien distinct pour ligature ou suture, et une fenêtre latérale traversante (228) par laquelle ledit berceau (224) débouche à l'extérieur du support, dans lequel ledit berceau (224) est formé d'un canal longitudinal qui débouche sur l'extrémité axiale proximale de la tige (220).

## Claims

1. A ligature and/or suture system for endoscopic surgery, comprising, in combination:
a tie (100) associated with a tubular endpiece (120) including a longitudinal channel and suitable for sliding therein; and
a rigid support (200) including a handle (210) secured to an elongate rod (220) which is provided at its proximal end (222) remote from the handle (210) with a reception cradle (224) suitable for removably receiving the tubular endpiece (120), and with a through lateral window (228) whereby said cradle (224) opens out to the outside of the support (200) to enable the tie (110) to pass therethrough such that traction exerted on an end (118) of the tie relative to the support (200) serves to tighten the ligature or the suture, the system being characterized by the facts that:
the tubular endpiece (120) is formed by a separate part, distinct from the tie (100); and
the reception cradle (224) is formed by a longitudinal channel that opens out to the proximal axial end of the rod (220) of the rigid support (200).

2. A system according to claim 1, characterized by the fact that the handle (210) of the support (200) is provided with a slideway (212) suitable for receiving in translation a cursor (140, 240) secured to the distal end (118) of the tie (110).

3. A system according to claim 2, characterized by the fact that the cursor (140) is permanently secured to the distal end (118) of the tie (110).

4. A system according to claim 2 or 3, characterized by the fact that the cursor (140) is packaged together with the tie (100) in sterile packaging.

5. A system according to claim 2, characterized by the fact that the cursor (240) is permanently mounted on the handle (210), which cursor (240) is adapted to receive the distal end (118) of the tie (110) in removable manner.

6. A system according to claim 5, characterized by the fact that the cursor (240) comprises a clip structure (242).

7. A system according to any one of claims 1 to 6, characterized by the fact the tie (100) is for single use only.

8. A system according to any one of claims 1 to 7, characterized by the fact that the support (200) is reusable.

9. A system according to any one of claims 1 to 8, characterized by the fact that the tubular endpiece (120) is wade of plastics material.

10. A system according to any one of claims 1 to 9, characterized by the fact that it comprises retaining means suitable for selectively securing the tubular endpiece (120) on the proximal end (222) of the rod (220) in the reception cradle (224) therefore.

11. A system according to claim 10, characterized by the fact that the retaining means comprise at least one annular groove (124) formed on the tubular endpiece (120).

12. A system according to any one of claims 1 to 11, characterized by the fact that the retaining means comprise two annular grooves (124, 127) formed on the tubular endpiece (120) respectively in the vicinity of its proximal end and of its distal end.

13. A system according to any one of claims 10 to 12 characterized by the fact that the retaining means comprise a rib (226) formed on the inside surface of the reception cradle (224).

14. A system according to any one of claims 1 to 13, characterized by the fact that the proximal end of the tubular endpiece (120) is tapering.

15. A system according to any one of claims 1 to 14, characterized by the fact that the inside diameter of the longitudinal channel through the tubular endpiece (120) is greater than the diameter of the filament (110) constituting the tie (100) while being less than the transverse size of a slip knot (116) formed on said tie (100).

16. A system according to any one of claims 1 to 15, characterized by the fact that the lateral window (228) extends over the entire length of the reception cradle (224).

17. A system according to any one of claims 1 to 16, characterized by the fact that the longitudinal axis of the reception cradle (224) is inclined relative to the longitudinal axis of the support rod (220).

18. A system according to any one of claims 1 to 16, characterized by the fact that the reception cradle (224) is hinged to the proximal end (222) of the support (200) and is associated with means for adjusting its orientation.

19. A system according to any one of claims 1 to 18, taken in combination with claim 2, characterized by the fact that the assembly comprising the slideway (212) an the cursor (140, 240) includes a groove having converging edges and a tenon having complementary right cross-section.

20. A system according to any one of claims 1 to 19, characterized by the fact that the tie (100) is selected from the group comprising: a ligature filament (110) provided at its proximal end with a preestablished loop (114) having a slip knot (116); a simple ligature filament (110); and a suture filament (110) provided with a needle at its proximal end.

21. A system according to any one of claims 1 to 20, characterized by the fact that the filament (110) of the tie (100) is provided with an auxiliary endpiece (130) at its distal end (118).

22. A system according to claim 21, characterized by the fact that the auxiliary endpiece (130) has a right cross-section that is greater than that of the channel passing through the tubular endpiece (120).

23. A system according to claim 21 or 22, characterized by the fact that the auxiliary endpiece (130) is shaped as a cursor which is adapted to be slidably guided in removable manner along the handle (210).

24. A system according to claim 21 or 22, characterized by the fact that the auxiliary endpiece (130) is adapted to be fixed on a cursor that is slidably mounted on the handle (210).

25. A system according to any one of claims 1 to 24, characterized by the fact that the tie (110) comprises a preformed loop (1100) formed on the length thereof between its distal end and the tubular endpiece (120).

26. A system according to any one of claims 1 to 25, characterized by the fact that the tubular endpiece (120) is integrally molded with an auxiliary endpiece (130) provided on the distal end of the tie, said tubular endpiece (120) and the auxiliary endpiece (130) being initially united via a zone of weakness (132).

27. A system according to any one of claims 1 to 26, characterized by the fact that the proximal end (126) of the tubular endpiece (120) has a surface in the form of half of a torus (129).

28. A ligature or suture tie for use in an endoscopic surgical operation and in accordance with any one of claims 1 to 27, characterized by the fact that it comprises a tie (100) and a tubular endpiece (120) provided with a longitudinal channel, formed by a separate part, distinct from the tie (100), which is slidably mounted in said longitudinal channel, and characterized by the fact that said tubular endpiece (120) includes means (124) for removably fixing to the end of a rod (220) of a rigid support (200).

29. A rigid support for an endoscopic surgical operation and in accordance with any one of claims 1 to 27, characterized by the fact that it comprises a handle (210) secured to an elongate rod (220), which rod is provided at its proximal end (222) remote from the handle (210) with a reception cradle (224) suitable for removably receiving a tubular endpiece (210) slidably receiving a distinct tie for ligature or suture purposes, and a through lateral window (228) whereby said cradle (224) opens out to the outside of the support in which said cradle (224) is formed by a longitudinal channel that opens out to the proximal axial end of the rod (220).

## Patentansprüche

1. Abbinde- und/oder Nähsystem für die endoskopische Chirurgie mit einer Kombination:
- eines Bandes (100), das mit einem rohrförmigen Ansatzstück (120) derart zusammengefügt ist, daß es von diesem umschlossen und in diesem verschiebbar ist sowie
- eines starren Trägers (200) mit einem Haltegriff (210), der fest mit einem langgestreckten dünnen Stab (220) verbunden ist, welcher im Bereich seines proximalen, von dem Griff (210) abgewandten Endes (222) einen Aufnahmehohlraum (224) aufweist, der das rohrförmige Ansatzstück (120) herausnehmbar aufzunehmen vermag, wobei ein seitliches Fenster (228) quer durch diesen Hohlraum (224) verläuft und denselben zur Außenseite des Trägers (200) hin freilegt, um den Durchtritt des Bandes (110) in der Weise zu ermöglichen, daß eine auf ein Ende (118) desselben im Verhältnis zum Träger (200) ausgeübte Zugkraft das Zusammenziehen der Abbinde- oder Nahtstelle sichert, dadurch gekennzeichnet, daß
- das rohrförmige Ansatzstück (120) als ein gesondertes, vom Band (100) getrenntes Teil ausgebildet ist, und
- der Aufnahmehohlraum (224) als Längskanal ausgebildet ist, der am axialen, proximalen Ende des dünnen Stabes (220) des starren Trägers (200) mündet.

2. System nach Anspruch 1, dadurch gekennzeichnet, daß der Haltegriff (210) des Trägers (200) mit einer Gleitführung (212) versehen ist, auf welcher die Längsbewegung eines Schiebers (140, 240) erfolgen kann, der am distalen Ende (118) des Bandes (110) angebracht ist.

3. System nach Anspruch 2, dadurch gekennzeichnet, daß der Schieber (140) dauerhaft am distalen Ende (118) des Bandes befestigt ist.

4. System nach einem der Ansprüche 2 oder 3, dadurch gekennzeichnet, daß der Schieber (140) zusammen mit dem Band (100) unter steriler Verpackung aufbewahrt wird.

5. System nach Anspruch 2, dadurch gekennzeichnet, daß der Schieber (240) dauerhaft am Haltegriff (210) befestigt ist, wobei dieser Schieber (240) derart eingerichtet ist, daß er das distale Ende (118) des Bandes (110) herausnehmbar aufnimmt.

6. System nach Anspruch 5, dadurch gekennzeichnet, daß der Schieber (240) eine Klammerstruktur (242) aufweist.

7. System nach einem der Ansprüche 1 bis 6, dadurch gekennzeichnet, daß, das Band (100) nur zum einmaligen Gebrauch bestimmt ist.

8. System nach einem der Ansprüche 1 bis 7, dadurch gekennzeichnet, daß der Träger (200) wiederverwendbar ist.

9. System nach einem der Ansprüche 1 bis 8, dadurch gekennzeichnet, daß das rohrförmige Ansatzstück (120) aus Plastikmaterial hergestellt ist.

10. System nach einem der Ansprüche 1 bis 9, dadurch gekennzeichnet, daß es eine Rückhalteinrichtung aufweist, die derart eingerichtet ist, daß sie das rohrförmige Ansatzstück (120) am proximalen Ende (222) des dünnen Stabes (220) in dessen Aufnahmehohlraum (222) wahlweise festhält.

11. System nach Anspruch 10, dadurch gekennzeichnet, daß die Rückhalteeinrichtung mindestens eine, auf dem rohrförmigen Ansatzstück (120) ausgebildete ringförmige Rille (124) aufweist.

12. System nach einem der Ansprüche 1 bis 11, dadurch gekennzeichnet, daß die Rückhalteeinrichtung zwei ringförmige Rillen (124, 127) aufweist, die auf dem rohrförmigen Ansatzstück (120) jeweils in der Nähe seines proximalen und seines distalen Endes ausgebildet sind.

13. System nach einem der Ansprüche 10 bis 12, dadurch gekennzeichnet, daß die Rückhalteeinrichtung eine auf der Innenfläche des Aufnahmehohlraums (224) ausgebildete Rippe (226) aufweist.

14. System nach einem der Ansprüche 1 bis 13, dadurch gekennzeichnet, daß das rohrförmige Ansatzstück (120) an seinem proximalen Ende (126) spitz ausgebildet ist.

15. System nach einem der Ansprüche 1 bis 14, dadurch gekennzeichnet, daß der Innendurchmesser des Längskanals des rohrförmigen Ansatzstückes (120) größer ist als der Durchmesser des Fadens (110), der Bestandteil des Bandes (100) ist, und beide kleiner sind als der Umriß in Querrichtung eines auf dem Band (100) ausgebildeten gleitenden Knotens (116).

16. System nach einem der Ansprüche 1 bis 15, dadurch gekennzeichnet, daß sich das seitliche Fenster (228) über die gesamte Länge des Aufnahmehohlraums (224) erstreckt.

17. System nach einem der Ansprüche 1 bis 16, dadurch gekennzeichnet, daß die Längsachse des Aufnahmehohlraums (224) in bezug auf die Längsachse des dünnen Trägerstabes (220) geneigt ist.

18. System nach einem der Ansprüche 1 bis 16, dadurch gekennzeichnet, daß der Aufnahmehohlraum (224) auf dem proximalen Ende (222) des Trägers gelenkig angebracht und mit einer Einrichtung zur Einstellung der Orientierung versehen ist.

19. System nach einem der Ansprüche 1 bis 18 in Kombination mit Anspruch 2, dadurch gekennzeichnet, daß die aus Gleitführung (212) und Schieber (140, 240) bestehende Baugruppe eine Nut mit konvergierenden Rändern und einen Zapfen mit genau komplementärem Querschnitt aufweist.

20. System nach einem der Ansprüche 1 bis 19, dadurch gekennzeichnet, daß das Band (100) aus einer Gruppe ausgewählt ist, welche umfaßt: einen Abbindefaden (110), der an seinem proximalen Ende eine vorgefertigte Schlinge (114) mit einem gleitenden Knoten (116) aufweist, einen einfachen Abbindefaden oder einen Nähfaden (110), der an seinem proximalen Ende mit einer Nadel versehen ist.

21. System nach einem der Ansprüche 1 bis 20, dadurch gekennzeichnet, daß der Faden (110) des Bandes (100) an seinem distalen Ende (118) mit einem Hilfs-Ansatzstück (130) versehen ist.

22. System nach Anspruch 21, dadurch gekennzeichnet, daß das Hilfs-Ansatzstück (130) einen Querschnitt hat, der etwas größer ist als derjenige des Kanals, der das rohrförmige Ansatzstück (120) durchläuft.

23. System nach einem der Ansprüche 21 oder 22, dadurch gekennzeichnet, daß das Hilfs-Ansatzstück (130) zugleich ein Schieber ist, der herausnehmbar im Haltegriff (210) gleitend geführt werden kann.

24. System nach einem der Ansprüche 21 oder 22, dadurch gekennzeichnet, daß das Hilfs-Ansatzstück (130) so eingerichtet ist, daß es auf einem Schieber befestigt werden kann, der gleitend auf dem Haltegriff (210) angebracht ist.

25. System nach einem der Ansprüche 1 bis 24, dadurch gekennzeichnet, daß das Band (110) auf seiner Länge zwischen seinem distalen Ende und dem rohrförmigen Ansatzstück (120) eine vorgeformte Schlinge (1100) aufweist.

26. System nach einem der Ansprüche 1 bis 25, dadurch gekennzeichnet, daß das rohrförmige Ansatzstück (120) zusammen mit einem für das distale Ende des Bandes vorgesehenen Hilfs-Ansatzstück (130) aus der Formgebung kommt, wobei das rohrförmige Ansatzstück (120) und das Hilfs-Ansatzstück (130) zunächst an einer Schwachstelle (132) verbunden sind.

27. System nach einem der Ansprüche 1 bis 26, dadurch gekennzeichnet, daß das proximale Ende (126) des rohrförmigen Ansatzstückes (120) eine halbringförmige Oberfläche (129) aufweist.

28. Band zum Abbinden oder Nähen bei einer endoskopischen chirurgischen Operation nach einem der Ansprüche 1 bis 27, dadurch gekennzeichnet, daß es ein Band (100) und ein rohrförmiges, mit einem Längskanal versehenes Ansatzstück (120) umfaßt, wobei letzteres als getrenntes, vom Band (100) unterscheidbares Teil geformt ist und das Band gleitend in dem Längskanal angebracht ist und weiterhin dadurch gekennzeichnet, daß das rohrförmige Ansatzstück (120) eine Einrichtung (124) zur herausnehmbaren Befestigung am Ende eines dünnen Stabes (220) eines starren Trägers (200) trägt.

29. Starrer Träger für eine chirurgische endoskopische Operation nach einem der Ansprüche 1 bis 27, dadurch gekennzeichnet, daß er einen Haltegriff (210) aufweist, der fest mit einem langgestreckten dünnen Stab (220) verbunden ist, welcher an seinem proximalen, von dem Griff (210) abgewandten Ende (222) einen Aufnahmehohlraum (224) aufweist, der ein rohrförmiges Ansatzstück (120) herausnehmbar aufzunehmen vermag, das ein gesondertes Band zum Abbinden oder Nähen gleitend aufnimmt, wobei ein seitliches Fenster (228) quer durch diesen Hohlraum (224) verläuft und denselben zur Außenseite des Trägers (200) hin freilegt, in welchem der Hohlraum (224) als ein Längskanal ausgebildet ist, der am proximalen, axialen Ende des dünnen Stabes (220) mündet.
